# EUROPÄISCHE PATENTSCHRIFT

(11) **EP 2 111 831 B1**
(45) Veröffentlichungstag und Bekanntmachung des Hinweises auf die Patenterteilung: **20.01.2010**
(21) Anmeldenummer: 08007790.2
(22) Anmeldetag: 22.04.2008
(51) Int. Cl.: A61F 9/01, A61B 3/107, A61B 3/113

(54) **Einrichtung fuer die laseroptische Augenchirurgie**
Device for laser optic eye surgery
Dispositif pour la chirurgie oculaire à laser optique

(43) Veröffentlichungstag der Anmeldung: 28.10.2009
(73) Patentinhaber: WaveLight AG, 91058 Erlangen (DE)
(72) Erfinder: Vogler, Klaus, Dr., 90542 Eckental (DE); Kittelmann, Olaf, Dr., 90408 Nuernberg (DE)
(74) Vertreter: Katérle, Axel

(56) Entgegenhaltungen:
- EP-A- 0 983 757
- DE-A1- 10 207 535
- DE-A1-102005 013 949

## Beschreibung

Die Erfindung betrifft eine Einrichtung für die laseroptische Augenchirurgie.

In der Augenchirurgie werden Laser vielfältig eingesetzt. Beispielsweise müssen bei der refraktiven Augenchirurgie, die der Behebung von Fehlsichtigkeiten des Auges dient, häufig Schnitte in die Hornhaut (Kornea) oder die Linse eingebracht werden. Eine verbreitete Technik hierbei ist die sogenannte femto-LASIK. Bei der LASIK (Laser In Situ Keratomileusis) wird zunächst ein oberflächliches Scheibchen aus der Hornhaut ausgeschnitten. Dieses Scheibchen, das in der Fachwelt als Flap bezeichnet wird, hängt noch in einem Scharnierbereich am übrigen Epithelgewebe; es wird zur Seite geklappt, um so die darunter liegenden Gewebebereiche der Hornhaut freizulegen. Sodann wird mittels eines Excimerlasers nach Maßgabe eines zuvor ermittelten Ablationsprofils Material vom Stroma abgetragen. Danach wird der Flap zurückgeklappt und verheilt in relativ kurzer Zeit mit dem übrigen Gewebe. Klassischerweise wird der Flap mechanisch mittels eines Mikrokeratoms erzeugt. Schonender ist jedoch eine Erzeugung mittels eines Lasers. Hierzu wird Laserstrahlung mit ultrakurzen Pulsdauern im Bereich von Femtosekunden eingesetzt (daher: femto-LASIK). Für eine präzise Lokalisierung des Schnitts ist ein vergleichsweise kleiner Fokusdurchmesser mit kurzer Rayleigh-Länge erforderlich. Typische Fokusdurchmesser bei der Einbringung von Flapschnitten oder anderen Inzisionen in der Hornhaut oder der Linse des Auges liegen bei etwa 5 µm oder darunter. Übliche Rayleigh-Längen liegen bei etwa 10 µm oder darunter.

Die Materialbeeinflussung und -veränderung findet im wesentlichen nur im Bereich des Strahlfokus statt. Außerhalb des Strahlfokus ist die Energiedichte zu gering. Wegen der kleinen Fokusabmessungen ist eine präzise Fokussierung des Laserstrahls auf den gewünschten Ort erforderlich, an dem ein Schnitt gesetzt werden soll. Die präzise Einstellung des Fokusorts in der x-y-Ebene (hierunter sei die Ebene senkrecht zur Strahlachse verstanden) gelingt mit einer Ablenkeinheit (Scanner) aus einem oder mehreren gesteuert verstellbaren Ablenkspiegeln. Probleme sind jedoch mit der Fokussteuerung in z-Richtung (d.h. in Richtung der Strahlachse) verbunden. Will man beispielsweise bei einem Flächenschnitt, der zumindest teilweise in konstanter Tiefe in der Hornhaut verlaufen soll (wie dies bei einem Flap der Fall ist), eine z-Verstellung des Strahlfokus vermeiden, muss man eine auf der augenzugewandten Seite flache Aplanationsplatte auf das Auge aufsetzen, um so die Hornhaut flachzudrücken Der Flap kann dann mittels eines ebenen Flächenschnitts erzeugt werden.

Die Aplanationsplatte wird dabei gegenüber dem die Laserstrahlung fokussierenden Objektiv fixiert und liefert so eine z-Referenz für den Strahlfokus. Leider erhöht sich aber durch das Flachdrücken des Auges der Augeninnendruck merklich, was unter Umständen sogar zu einer irreversiblen Schädigung des Sehnervs führen kann.

Geringere Verformungen des Auges sind möglich, wenn ein auf seiner augenzugewandten Seite konkav geformtes Kontaktglas verwendet wird. Allerdings sind auch bei solchen Linsen Verformungen des Auges niemals vollständig zu vermeiden. Außerdem haben gewölbte Kontaktgläser regelmäßig einen negativen Einfluss auf die Qualität des Strahlfokus. Die gekrümmte Grenzfläche zwischen Kontaktglas und Hornhaut kann beispielsweise zu Koma-Verzeichnungen führen, die sich ihrerseits auf die Qualität der Inzision ungünstig auswirken können.

EP 0 983 757 A2 offenbart eine Lasereinrichtung für die Ablation von kornealem Gewebe eines Auges. Die Einrichtung umfasst zwei verschiedene Laserquellen, deren eine zur Vermessung der kornealen Oberfläche und deren andere zur Gewebeablation dient. Die optischen Strahlungspfade beider Laserquellen laufen zusammen und enthalten in ihrem gemeinsamen Abschnitt eine längsverschieblich angeordnete Fokussierlinse, deren Längsverstellbarkeit zur Fokuseinstellung bei der Oberflächenvermessung der Kornea genutzt wird. Der für die Ablation verwendete Arbeitslaser ist ein bei 193 nm strahlender Excimer-Laser.

DE 10 2005 013 949 A1 offenbart in seinen Fig. 1-3 Ausführungsformen eines Lasersystems für die Behandlung des menschlichen Auges mit gepulster Femtosekunden-Laserstrahlung. Das System weist eine Strahlaufweitungsoptik mit einer Zerstreuungslinse und einer anschließenden Sammellinse auf, wobei die Zerstreuungslinse in Strahlrichtung mittels eines Stellantriebs verschiebbar ist, um die Fokuslage der Laserstrahlung in z-Richtung zu justieren. Die z-Lage des Strahlfokus ist gegenüber einem Kontaktglas referenziert, das auf das zu behandelnde Auge aufzusetzen ist.

DE 102 07 535 A1 offenbart eine Laserbearbeitungsvorrichtung, die das Laserlicht einer einzelnen Laserquelle wahlweise als Messstrahlung und als Bearbeitungsstrahlung ausgeben kann.

Aufgabe der Erfindung ist es, eine Einrichtung für die laseroptische Augenchirurgie zu schaffen, die eine schonende, dennoch präzise Behandlung des Auges gestattet.

Bei der Lösung dieser Aufgabe geht die Erfindung aus von einer Einrichtung für die laseroptische Augenchirurgie, mit einer Quelle gepulster Femtosekunden-Laserstrahlung sowie optischen Komponenten zur Führung der Laserstrahlung und Fokussierung derselben auf einen Behandlungsort am oder im Auge, wobei die optischen Komponenten eine Mehrzahl im Strahlengang der Laserstrahlung hintereinander angeordneter Linsen umfassen. Erfindungsgemäß ist nach einer Betrachtungsweise wenigstens eine der Linsen in Richtung des Strahlengangs relativ zu anderen Linsen verstellbar angeordnet, wobei der verstellbaren Linse zu ihrer Verstellung eine Stellanordnung zugeordnet ist und zur Steuerung der Stellanordnung eine Steuereinheit vorgesehen ist, welche dazu eingerichtet ist, auf Messdaten über die Topographie einer Fläche des Auges zuzugreifen und die Stellanordnung abhängig von der gemessenen Flächentopographie zu steuern.

Die erfindungsgemäße Lösung basiert auf einer z-Steuerung des Strahlfokus abhängig von einer gemessenen Flächentopographie des Auges. Sie gestattet es, auf ein auf das Auge aufgesetztes Kontaktglas zu verzichten, sei es in Form einer planen Aplanationsplatte oder in Form einer konkav gewölbten Linse. Der vollständige Verzicht auf ein solches Kontaktglas hat dementsprechend zur Folge, dass keinerlei unerwünschte Verformungen des Auges bei der Behandlung auftreten, noch optische Verzerrungen aufgrund des Kontaktglases. Insbesondere repräsentieren die Topographiemessdaten die Topographie der äußeren Hornhautoberfläche. Es versteht sich jedoch, dass es grundsätzlich denkbar ist, eine andere Fläche innerhalb des Auges als zu vermessende Referenzfläche zu verwenden, beispielsweise die Linsenoberfläche.

Die Topographie der Fläche des Auges kann beispielsweise mit Lichtspalttechnik, mittels Ultraschall oder mittels optischer Kohärenztomographie gemessen werden. Diese Techniken sind in der Fachwelt an sich bekannt, weshalb es hier keiner weiteren Erläuterungen zur Art der Gewinnung der Topographiemessdaten bedarf. Eine nach einem oder mehreren der genannten Messprinzipien arbeitende Messanordnung kann Teil der erfindungsgemäßen Einrichtung sein und ihre Messdaten in einem Speicher ablegen, auf den die Steuereinheit Zugriff hat.

Soweit für die Topographiemessung auf optische Kohärenztomographie zurückgegriffen wird, lehrt die Erfindung insbesondere den Einsatz extrem schneller Einrichtungen für die optische Kohärenztomographie unter Einsatz von Femtosekunden-Strahlungsquellen, bevorzugt mit Repetitionsraten im Bereich von 10 GHz und bevorzugt im Bereich von 100 GHz oder mehr, beispielsweise den Einsatz sogenannter VECSELs (Vertical External Cavity Surface Emitting Laser). Solche Halbleiter-Laserdioden können elektrisch oder optisch gepumpt werden und erreichen trotz einer Baugröße im Zentimeterbereich sehr hohe Leistungen und Wirkungsgrade. Auch können Femtosekunden-Faserlaser im Rahmen der optischen Kohärenztomographie eingesetzt werden. Solche Strahlungsquellen können Fs-Superkontinua mit Bandbreiten größer 100 nm bis zu 1000 nm und mit Repetitionsraten größer 100 GHz erzeugen, so dass eine extrem hohe Messrate erreichbar ist, die bei Bedarf eine quasi Echtzeit-Messung der Topographie der Referenzfläche (zum Beispiel Hornhautoberfläche) während des Operationsvorgangs gestattet. Es muss demnach nicht notwendig die Topographiemessung vollständig vor der Operation durchgeführt werden, sondern kann während der Operation sozusagen "online" durchgeführt werden.

Zweckmäßigerweise bilden die optischen Komponenten der erfindungsgemäßen Einrichtung eine Strahlaufweitungsoptik, eine in Richtung des Strahlengangs hinter der Strahlaufweitungsoptik angeordnete, der Strahlablenkung in einer Ebene quer zur Strahlrichtung dienende Ablenkeinheit sowie eine in Richtung des Strahlengangs hinter der Ablenkeinheit angeordnete Fokussieroptik. Die Strahlaufweitungsoptik weitet den Laserstrahl hinreichend auf, um die für die angestrebten kleinen Fokusdurchmesser benötigte hohe numerische Apertur der Fokussieroptik zu erzielen. Regelmäßig wird die Strahlaufweitungsoptik mehrere in Strahlrichtung hintereinander angeordnete Linsen aufweisen, von denen zumindest eine als Streulinse ausgebildet ist und wenigstens eine andere als Sammellinse ausgebildet ist, wobei die Streulinse vor der Sammellinse liegt. Marktübliche Strahlaufweitungsoptiken bestehen in der Regel aus insgesamt zwei oder drei Linsen, von denen die erste Linse (Eingangslinse) stets eine Streulinse ist. Ihr Durchmesser ist wesentlich kleiner als der der anschließenden Sammellinse(n). Dementsprechend ist auch ihre Masse regelmäßig beträchtlich geringer als die der anschließenden Sammellinse(n) der Strahlaufweitungsoptik. Aus diesem Grund sieht eine bevorzugte Ausführungsform der Erfindung vor, eine Streulinse der Strahlaufweitungsoptik, insbesondere die Eingangslinse der Strahlaufweitungsoptik, verstellbar anzuordnen und sie für den Zweck der z-Steuerung des Strahlfokus relativ zu wenigstens einer Sammellinse der Strahlaufweitungsoptik in Strahlrichtung zu verschieben. Die geringe Masse der Streulinse erlaubt dabei eine hochdynamische Verstellung derselben, beispielsweise mittels eines elektromotorischen oder piezoelektrischen Stellantriebs. Dagegen wäre im Fall einer Verstellung der anschließenden Sammellinse oder sogar der Fokussieroptik die zu bewegende Masse ungleich größer, was der gewünschten Dynamik abträglich wäre.

Es hat sich gezeigt, dass bei geeigneter Auslegung und Positionierung der Linsen der erfindungsgemäßen Einrichtung ein Verstellweg der Eingangslinse der Strahlaufweitungsoptik von 10,0 mm ausreichen kann, um den Strahlfokus in einem Bereich von 1,4 mm verschieben zu können. Dies ist in der Regel ausreichend, um die Hornhautwölbung auszugleichen und einen in konstanter Tiefe liegenden Flächenschnitt in die Hornhaut einzubringen.

Die Steuereinheit kann dazu eingerichtet sein, eine Soll-Position für die verstellbare Linse abhängig von der gemessenen Flächentopographie sowie abhängig vom Höhenabstand eines gewünschten Einwirkorts der Strahlung im Auge von der topographisch vermessenen Fläche zu ermitteln und die Stellanordnung abhängig von der ermittelten Soll-Position zu steuern. Der Höhenabstand bezieht sich hierbei auf den Abstand in z-Richtung. Selbst bei vollkommen ruhiger Kopfhaltung und selbst bei Fixierung des Auges mittels eines Saugrings können geringfügige Bewegungen der Hornhaut in z-Richtung nicht vollständig vermieden werden. Solche Bewegungen sind zum Beispiel atmungsbedingt. Um dennoch den Strahlfokus stets präzise an der gewünschten Stelle des Auges positionieren zu können, ist die erfindungsgemäße Einrichtung bei einer bevorzugten Weiterbildung mit einer Messanordnung ausgestattet, welche dazu eingerichtet ist, Verlagerungen der Höhenposition wenigstens eines Referenzorts am oder im Auge zu erfassen. Die Steuereinheit ist dabei dazu eingerichtet, die ermittelte Soll-Position der verstellbaren Linse abhängig von der erfassten aktuellen Höhenposition des wenigstens einen Referenzorts zu korrigieren und die Stellanordnung abhängig von der korrigierten Soll-Position zu steuern. Als Referenzort kommt beispielsweise der Hornhautscheitelpunkt in Frage.

Im Fall, dass auf einen Saugring zur Fixierung des Auges verzichtet wird, sind selbst bei ansonsten ruhiger Kopfhaltung Drehbewegungen des Augapfels regelmäßig unvermeidbar. Auch solche Bewegungen des Auges können eine z-Korrektur der ermittelten Soll-Position für die verstellbare Linse erforderlich machen. Denn eine Drehung des Augapfels kann gleichzeitig eine Verschiebung der z-Koordinate eines gewünschten Einwirkorts der Laserstrahlung im Auge bewirken. Deshalb kann die erfindungemäße Einrichtung eine Messanordnung umfassen, welche dazu eingerichtet ist, Bewegungen mindestens eines Referenzorts am oder im Auge in einer Ebene quer zur Richtung des Strahlengangs zu erfassen, wobei die Steuereinheit dazu eingerichtet ist, die Soll-Position der verstellbaren Linse abhängig von der erfassten aktuellen Querposition des wenigstens einen Referenzorts zu korrigieren und die Stellanordnung abhängig von der korrigierten Soll-Position zu steuern.

Unabhängig davon, ob Drehbewegungen des Auges bei der Korrektur der Soll-Position der verstellbaren Linse berücksichtigt werden oder nicht, ist in jedem Fall eine Steuerung der Strahlablenkeinheit (Scanner) abhängig von den Augenbewegungen erforderlich, um den Strahlfokus stets präzise nachführen zu können. Hierfür geeignete Überwachungssysteme (Eyetracker") sind in der Fachwelt an sich bekannt. Beispielsweise kann hierbei der Hornhautscheitelpunkt auf Verlagerungen quer zur Strahlachse überwacht werden.

Bevorzugt ist der Fokusdurchmesser der Laserstrahlung nicht größer als etwa 10 µm, besser nicht größer als etwa 7 µm und noch besser nicht größer als etwa 5 µm. Die Rayleigh-Länge der Laserstrahlung ist bevorzugt nicht größer als etwa 20 µm, besser nicht größer als etwa 15 µm und noch besser nicht größer als etwa 10 µm.

Für die Erzeugung eines zur Hornhautoberfläche im wesentlichen parallelen Flächenschnitts in der Kornea durch Linienabtastung kann die Steuereinheit dazu eingerichtet sein, ein Steuersignal mit annähernder Dreieckscharakteristik und variierender Dreieckshöhe an die Stellanordnung zu liefern. Alternativ zu einer Linienabtastung, bei welcher der Strahl in parallelen Linien über das Auge bewegt wird, ist eine Spiralabtastung denkbar. Hierbei kann die Steuereinheit dazu eingerichtet sein, für die Erzeugung eines zur Hornhautoberfläche im wesentlichen parallelen Flächenschnitts in der Kornea durch Spiralabtastung ein Steuersignal monoton veränderlicher Amplitude an die Stellanordnung zu liefern. Die Dreiecksform des Steuersignals bei Linienabtastung hängt mit Tatsache zusammen, dass jede Linie vom tieferliegenden Hornhaut über höherliegende Zwischenbereiche und wieder zum Hornhautrand verläuft. Entsprechend ist die Linse in unterschiedliche Positionen einzustellen. Die variierende Dreieckshöhe des Steuersignals kommt daher, dass bei Linien, die über den Hornhautscheitelpunkt oder nahe desselben verlaufen, wegen der Hornhautwölbung der z-Hub der Linien größer ist als bei randnahen Linien. Bei Spiralabtastung dagegen ist ein stetige Verstellung der verstellbaren Linse in einer Richtung erforderlich, was sich in der monoton veränderlichen Amplitude des Steuersignals ausdrückt.

Die erfindungsgemäße Einrichtung benötigt nicht nur kein auf das Auge aufzusetzendes Kontaktglas, sie ist vorzugsweise auch frei von Anbringungsformationen für ein solches Kontaktglas.

Nach einem weiteren Gesichtpunkt sieht die Erfindung ein Steuerverfahren für eine Einrichtung für die laseroptische Augenchirurgie vor, wobei die Einrichtung eine Quelle gepulster Femtosekunden-Laserstrahlung, eine Mehrzahl im Strahlengang der Laserstrahlung hintereinander angeordneter Linsen, von denen wenigstens eine in Richtung des Strahlengangs relativ zu anderen Linsen verstellbar angeordnet ist, sowie eine Stellanordnung zur Verstellung der wenigstens einen verstellbaren Linse umfasst. Erfindungsgemäß wird bei dem Verfahren auf Grundlage gespeicherter Topographiemessdaten eine Soll-Position für die verstellbare Linse ermittelt und abhängig von der ermittelten Soll-Position ein Steuersignal für die Stellanordnung erzeugt.

Die Erfindung wird nachfolgend anhand der beigefügten Zeichnungen weiter erläutert. Es zeigen:
Figur 1 eine schematische Blockdarstellung eines Ausführungsbeispiels einer Einrichtung für die laseroptische Augenchirurgie,
Figur 2 einen qualitativen Verlauf der Stellposition einer einzeln verstellbaren Linse der Lasereinrichtung der Fig. 1 im Fall eines Linienscans und
Figur 3 einen qualitativen Verlauf der Stellposition der verstellbaren Linse im Fall eines Spiralscans.

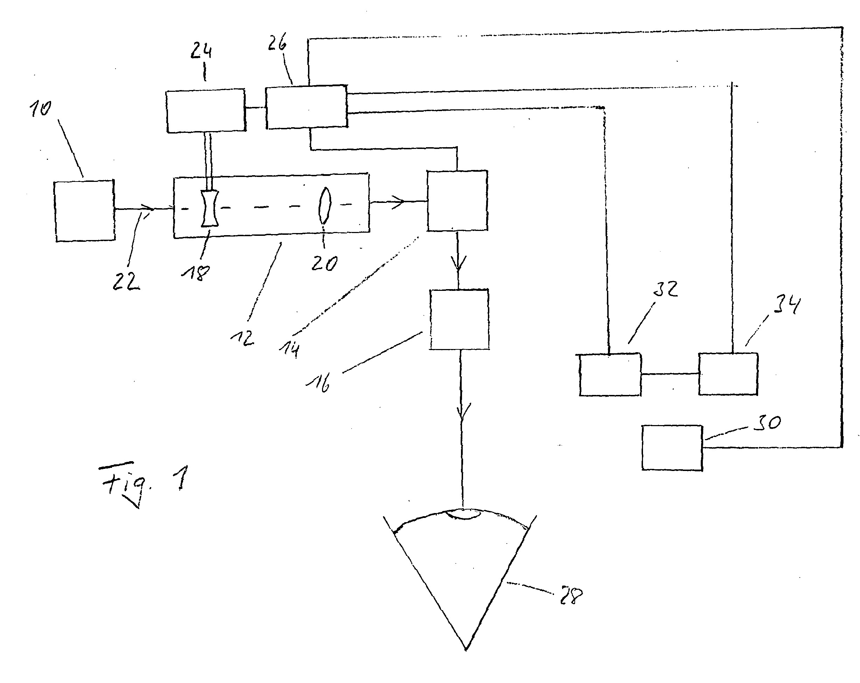

Die in Figur 1 gezeigte Lasereinrichtung für die Augenchirurgie umfasst einen Lasergenerator 10, der gepulste Laserstrahlung mit einer Pulsdauer im Bereich von Femtosekunden erzeugt und ausgibt. Der Begriff Femtosekunden ist hier breit zu verstehen; er soll nicht im Sinne einer scharfen Abgrenzung gegenüber Pulsdauern ab 1 ps verstanden werden. Ganz im Gegenteil, die Erfindung ist prinzipiell auch für Pulsdauern länger als 1 ps geeignet. Der Hinweis auf eine Pulsdauer im fs-Bereich ist allein insoweit richtungweisend, als bei der Augenchirurgie eingesetzte fs-Laser üblicherweise vergleichsweise kleine Fokusabmessungen mit einem Fokusdurchmesser von beispielsweise höchstens 5 µm und einer Rayleigh-Lange von höchstens 10 µm besitzen und die Erfindung besonders bei derart kleinen Fokusmaßen ihre Vorteile entfaltet. Nichtsdestotrotz liegt die Pulsdauer der Laserstrahlung vorzugsweise unterhalb 1 ps, z.B. im Bereich dreistelliger Femtosekunden.

Die Pulswiederholrate des Lasergenerators 10 kann beispielsweise im zwei- oder dreistelligen kHz-Bereich bis hin in den MHz-Bereich liegen. Insbesondere kann die Pulsrate des Lasergenerators 10 steuerbar sein. Die Wellenlänge der erzeugten und zur Behandlung eingesetzten Laserstrahlung kann beispielsweise im Infrarot-Bereich liegen, etwa um 1 µm herum, sie kann aber auch kürzer sein bis hinunter in den UV-Bereich.

Im Strahlengang des vom Lasergenerator ausgegebenen Laserstrahls folgen nacheinander eine Strahlaufweitungsoptik 12, ein Scanner 14 sowie eine Fokussieroptik 16. Die Strahlaufweitungsoptik 12 ist hier als Zweilinsen-System mit einer Streulinse 18 und einer dahinter liegenden Sammellinse 20 dargestellt. Es versteht sich, dass auch Strahlaufweitungsoptiken mit mehr als zwei Linsen verwendet werden können. Regelmäßig ist jedoch die Eingangslinse der Strahlaufweitungsoptik, hier die Linse 12, eine Streulinse. Die Linsen 18, 20 der Strahlaufweitungsoptik 12 sind in einem nicht näher dargestellten Gehäuse aufgenommen, wobei die Sammellinse 20 fest in dem Gehäuse angeordnet ist, die Streulinse 18 jedoch in Richtung der Strahlachse (bezeichnet mit 22) relativ zu der Sammellinse 20 verstellbar ist. Zur Verstellung der Streulinse 18 dient ein Stellantrieb 24, der von einer Steuereinheit 26 gesteuert wird. Beispielsweise ist der Stellantrieb 24 ein elektromotorischer oder piezoelektrischer Stellantrieb. Der Stellantrieb 24 greift beispielsweise in nicht näher dargestellter Weise an einer Linsenfassung an, die ihrerseits beweglich in dem Gehäuse geführt ist und die Streulinse 18 trägt.

Der Bewegungshub der Streulinse 18 in Richtung der Strahlachse 22 beträgt einige Millimeter, beispielsweise etwa 10 mm. Die benötigte Verstellgeschwindigkeit der Streulinse 18 kann unter anderem vom Abtastmuster abhängen, mit welchem der Laserstrahl über das zu behandelnde Auge - bezeichnet mit 28 - geführt wird. Es hat sich gezeigt, dass mit einer Verstellgeschwindigkeit der Streulinse 18 von mindestens 0,5 m/s, besser etwa 1 m/s, ein Flapschnitt in akzeptabel kurzer Zeit in die Hornhaut eingebracht werden kann. Der Stellantrieb 24 ist so ausgelegt, dass er diese Verstellgeschwindigkeit der Streulinse 18 gewährleisten kann.

Der Scanner 14 kann in an sich bekannter, hier nicht näher dargestellter Weise ein Paar Ablenkspiegel enthalten, die eine gezielte Ablenkung des Laserstrahls in einer senkrecht zur Strahlachse 22 liegenden x-y-Ebene ermöglichen. Er wird von der Steuereinheit 26 abhängig vom x-y-Bild der in das Auge 28 einzubringenden Inzision sowie abhängig von etwaigen Augenbewegungen gesteuert. Solche Augenbewegungen, die jedenfalls bei fehlender Fixierung des Augapfels mittels eines Saugrings unvermeidlich sind, können mittels eines schematisch als Funktionsblock 30 angedeuteten, mit der Steuereinheit 26 verbundenen Augenverfolgungssystems (Eyetracker) erfasst werden. Derartige Systeme sind in der Fachwelt an sich bekannt; auf nähere Erläuterungen ihrer Funktion und ihres Aufbaus kann deshalb hier verzichtet werden. Es genügt der Hinweis, dass der Eyetracker 30 Augenbewegungen beispielsweise anhand einer Mustererkennung erfassen kann, die er an einer Anzahl in schneller Reihe nacheinander aufgenommener Bilder der Pupille oder eines anderen Augenteils durchführt.

Die Fokussieroptik 16 ist in ebenfalls an sich bekannter Weise aus einer Mehrzahl hier nicht näher dargestellter Linsen aufgebaut. Die Brennweite der Fokussieroptik 16 ist fest. Die Fokussieroptik 16 kann unbeweglich in die Lasereinrichtung eingebaut sein, so dass eine z-Verstellung des Strahlfokus allein über eine Verstellung der Streulinse 18 möglich ist. Es ist freilich ebenso möglich, dass die Fokussieroptik 16 längs der Strahlachse 22 verstellbar angeordnet ist, so dass eine z-Verstellung des Strahlfokus sowohl über eine Verstellung der Streulinse 18 als auch über eine Verstellung der Fokussieroptik 16 möglich ist. Im letzteren Fall kann die Verstellbarkeit der Fokussieroptik 16 beispielsweise zur Grobeinstellung vor Beginn der eigentlichen Operation genutzt werden, während die Verstellbarkeit der Streulinse 18 für die Einstellung des Strahlfokus in unterschiedliche z-Positionen während der Behandlung genutzt wird. Zweckmäßigerweise wird bei der Grobeinstellung die Streulinse 18 in einer Mittenstellung gehalten, so dass sie anschließend bei der Operation in beide Verstellrichtungen ausreichend Bewegungshub bietet.

Die Lasereinrichtung nach Fig. 1 umfasst ferner eine Messanordnung 32, mit der sich die Topographie der Hornhautoberfläche des Auges 28 vermessen lässt. Beispielsweise arbeitet die Messanordnung nach dem Prinzip der optischen Kohärenztomographie (engl.: optical coherence tomography, kurz: OCT). Geeignete Auswertemittel innerhalb der Messanordnung 32 erzeugen aus den gemessenen Werten Topographiemessdaten, welche für das topographische Profil der Hornhautoberfläche repräsentativ sind, und stellen die Topographiemessdaten der Steuereinheit 26 zur Verfügung. Beispielsweise kann die Messanordnung 32 die Topographiemessdaten in einen Speicher 34 schreiben, aus dem sie die Steuereinheit 26 später abholen kann. Dies ermöglicht eine zeitlich entkoppelte Vermessung der gesamten Hornhauttopographie vor der eigentlichen Operation. Basierend auf den Topographiemessdaten kann die Steuereinheit dann zunächst ein zweidimensionales Stellprofil für die Streulinse 18 berechnen, das für alle Abtastpunkte in der x-y-Ebene jeweils eine Soll-Position angibt, in welche die Streulinse 18 einzustellen ist. Bei der Berechnung dieses Stellprofils berücksichtigt die Steuereinheit 26, in welchem Abstand in z-richtung (Höhenabstand) der Schnitt an jedem Punkt in der x-y-Ebene von der Hornhautoberfläche angebracht werden soll. Im Fall der Erzeugung eines Hornhautflaps beispielsweise wird üblicherweise eine konstante Dicke des Flaps angestrebt. Deshalb wird die Soll-Position der Streulinse 18 so berechnet, dass der Strahlfokus für alle x-y-Positionen des zu erzeugenden Flaps stets im wesentlichen den gleichen z-Abstand von der Hornhautoberfläche hat (abgesehen von den Flaprändern, wo der Schnitt zur Hornhautoberfläche geführt werden muss. Während der Operation genügt es dann, die z-Position des Hornhautscheitelpunkts oder/und wenigstens eines anderen Referenzpunkts des Auges 28 zu überwachen und die sich aus dem Stellprofil ergebende Soll-Position der Streulinse 18 abhängig von der aktuell erfassten z-Position des Augenreferenzorts zu korrigieren. Diese Überwachung kann ggf. ebenfalls von der OCT-Messanordnung 32 vorgenommen werden, die ihre diesbezüglichen Messwerte dann direkt an die Steuereinheit 26 liefert.

Das Auge 28 wird während der Behandlung überhaupt nicht oder nur mit einem Saugring fixiert, der Drehbewegungen des Augapfels verhindert. Wird ein Saugring verwendet, so ist dieser zweckmäßigerweise über eine geeignete mechanische Schnittstelle in z-Richtung fest an die Fokussieroptik 16 angekoppelt. In jedem Fall erfolgt die Behandlung ohne ein auf das Auge 28 aufgesetztes Kontaktglas.

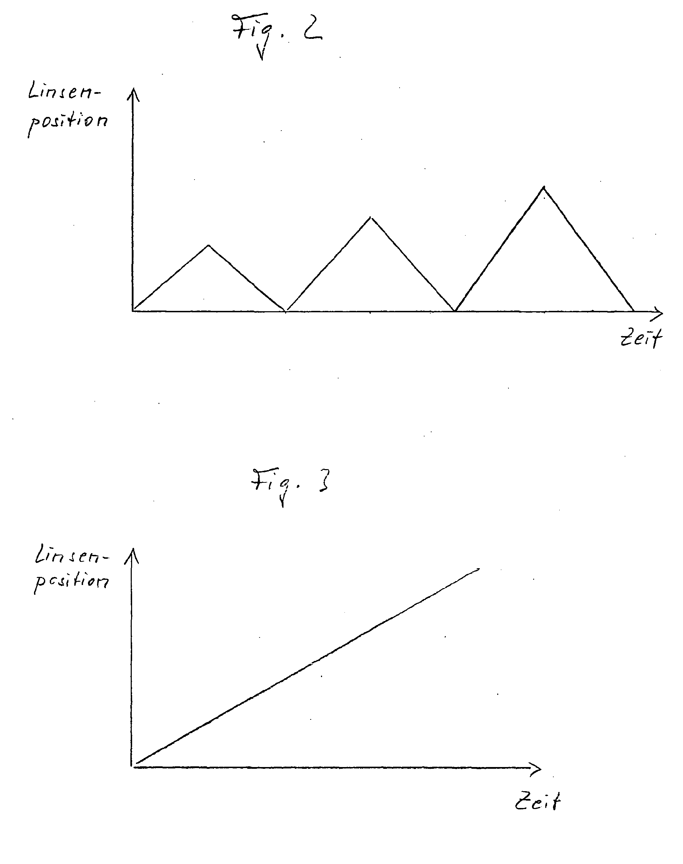

Für die Erzeugung eines Flächenschnitts in der Hornhaut sind sowohl eine Linienabtastung (Linescan) als auch eine Spiralabtastung (Spiralscan) bekannt. Die Fig. 2 und 3 zeigen typische, wenngleich idealisierte Verläufe der Stellposition der Streulinse 18 bei Erzeugung eines Hornhautflaps - Fig. 2 für einen Linienscan und Fig. 3 für einen Spiralscan. Im Fall des Linienscans, wo der Laserstrahl in nebeneinanderliegenden Linien über die Hornhaut geführt wird, wird die Streulinse 18 fortlaufend vor und zurück bewegt, um die bei jeder Linie zu überwindende Wölbung der Hornhaut zu berücksichtigen. Dies führt zu dem gezeigten dreiecksförmigen Verlauf der Stellposition. Entsprechend hat im Fall des Linienscans das von der Steuereinheit 26 an den Stellantrieb 24 gelieferte Steuersignal einen Dreieckscharakter. Da der Höhenunterschied zwischen Linienmitte und Linienende bei zentralen Scanlinien, die über die Mitte der Hornhaut verlaufen, größer ist als bei randnahen Scanlinien, variiert die Dreieckshöhe des Steuersignals.

Im Fall des Spiralscans dagegen genügt eine stetige Verstellung der Streulinse 18 in einer Richtung, gleichgültig, ob die Spirale vom Hornhautzentrum ausgeht oder vom Rand. Dementsprechend ergibt sich der in Fig. 3 gezeigte Verlauf der Linsenposition in Form einer monoton steigenden Gerade. Das an den Stellantrieb 24 gelieferte Steuersignal wird dementsprechend einen ähnlichen Charakter haben. Da beim Spiralscan geringere Höhendifferenzen pro Zeiteinheit zu überwinden sind, ermöglicht der Spiralscan geringere Verfahrgeschwindigkeiten der Streulinse 18 als der Linienscan. Andererseits ist beim Spiralscan zu berücksichtigen, dass für einen konstanten Abstand aufeinanderfolgender Schnittpunkte die Pulsrate des Lasergenerators 10 für äußere, randnahe Spiralwindungen größer als für innere, zentrale Spiralwindungen eingestellt werden muss, vorausgesetzt die Winkelgeschwindigkeit des Laserstrahls bleibt unverändert.

## Patentansprüche

1. Einrichtung für die laseroptische Augenchirurgie, mit einer Quelle (10) gepulster Femtosekunden-Laserstrahlung sowie optischen Komponenten (12, 14, 16) zur Führung der Laserstrahlung und Fokussierung derselben auf einen Behandlungsort am oder im Auge, wobei die optischen Komponenten eine Mehrzahl im Strahlengang der Laserstrahlung hintereinander angeordneter Linsen (18, 20) umfassen,
wobei wenigstens eine (18) der Linsen in Richtung des Strahlengangs relativ zu anderen Linsen verstellbar angeordnet ist, **dadurch gekennzeichnet, dass** der verstellbaren Linse zu ihrer Verstellung eine Stellanordnung (24) zugeordnet ist und dass zur Steuerung der Stellanordnung eine Steuereinheit (26) vorgesehen ist, welche dazu eingerichtet ist, auf Messdaten über die Topographie einer Fläche des Auges zuzugreifen und die Stellanordnung abhängig von der gemessenen Flächentopographie zu steuern.

2. Einrichtung nach Anspruch 1, **dadurch gekennzeichnet, dass** die wenigstens eine verstellbare Linse (18) Teil einer Strahlaufweitungsoptik (12) ist, welche in Strahlrichtung vor einer die Laserstrahlung in einer Ebene quer zur Strahlrichtung ablenkenden Ablenkeinheit (14) liegt.

3. Einrichtung nach Anspruch 2, **dadurch gekennzeichnet, dass** die Strahlaufweitungsoptik (12) zumindest eine Streulinse (18) und eine in Strahlrichtung dahinter liegende Sammellinse (20) aufweist, wobei die Streulinse mittels der Stellanordnung (24) relativ zu der Sammellinse verstellbar ist.

4. Einrichtung nach einem der Ansprüche 1 bis 3, **dadurch gekennzeichnet, dass** die Steuereinheit (26) dazu eingerichtet ist, eine Soll-Position für die verstellbare Linse (18) abhängig von der gemessenen Flächentopographie sowie abhängig vom Höhenabstand eines gewünschten Einwirkorts der Strahlung im Auge von der topographisch vermessenen Fläche zu ermitteln und die Stellanordnung (24) abhängig von der ermittelten Soll-Position zu steuern.

5. Einrichtung nach Anspruch 4, **gekennzeichnet durch** eine Messanordnung (32), welche dazu eingerichtet ist, Verlagerungen der Höhenposition wenigstens eines Referenzorts am oder im Auge zu erfassen, wobei die Steuereinheit (26) dazu eingerichtet ist, die Soll-Position der verstellbaren Linse (18) abhängig von der erfassten aktuellen Höhenposition des wenigstens einen Referenzorts zu korrigieren und die Stellanordnung (24) abhängig von der korrigierten Soll-Position zu steuern.

6. Einrichtung nach Anspruch 4 oder 5, **gekennzeichnet durch** eine Messanordnung (32), welche dazu eingerichtet ist, Bewegungen mindestens eines Referenzorts am oder im Auge (28) in einer Ebene quer zur Richtung des Strahlengangs zu erfassen, wobei die Steuereinheit (26) dazu eingerichtet ist, die Soll-Position der verstellbaren Linse (18) abhängig von der erfassten aktuellen Querposition des wenigstens einen Referenzorts zu korrigieren und die Stellanordnung (24) abhängig von der korrigierten Soll-Position zu steuern.

7. Einrichtung nach einem der Ansprüche 1 bis 6, **dadurch gekennzeichnet, dass** die Einrichtung frei von einem auf die Hornhaut aufzusetzenden Kontaktglas sowie frei von Anbringungsformationen für ein solches Kontaktglas ist.

8. Einrichtung nach einem der Ansprüche 1 bis 7, **gekennzeichnet durch** eine Messanordnung (32), welche dazu eingerichtet ist, die Hornhautoberfläche des Auges topographisch zu vermessen.

9. Einrichtung nach einem der Ansprüche 1 bis 8, **dadurch gekennzeichnet, dass** der Fokusdurchmesser der Laserstrahlung nicht größer als etwa 10 µm, vorzugsweise nicht größer als etwa 7 µm und höchstvorzugsweise nicht größer als etwa 5 µm ist und dass die Rayleigh-Länge der Laserstrahlung nicht größer als etwa 20 µm, vorzugsweise nicht größer als etwa 15 µm und höchstvorzugsweise nicht größer als etwa 10 µm ist.

10. Einrichtung nach einem der Ansprüche 1 bis 9, **dadurch gekennzeichnet, dass** die Steuereinheit (26) dazu eingerichtet ist, für die Erzeugung eines zur Hornhautoberfläche im wesentlichen parallelen Flächenschnitts in der Kornea durch Linienabtastung ein Steuersignal mit annähernder Dreieckscharakteristik und variierender Dreieckshöhe an die Stellanordnung zu liefern.

11. Einrichtung nach einem der Ansprüche 1 bis 10, **dadurch gekennzeichnet, dass** die Steuereinheit (26) dazu eingerichtet ist, für die Erzeugung eines zur Hornhautoberfläche im wesentlichen parallelen Flächenschnitts in der Kornea durch Spiralabtastung ein Steuersignal monoton veränderlicher Amplitude an die Stellanordnung zu liefern.

12. Einrichtung nach einem der Ansprüche 1 bis 11,
**dadurch gekennzeichnet, dass** die optischen Komponenten eine Strahlaufweitungsoptik (12), eine in Richtung des Strahlengangs hinter der Strahlaufweitungsoptik angeordnete, der Strahlablenkung in einer Ebene quer zur Strahlrichtung dienende Ablenkeinheit (14) sowie eine in Richtung des Strahlengangs hinter der Ablenkeinheit angeordnete Fokussieroptik (16) bilden, dass die Strahlaufweitungsoptik (12) mehrere in Strahlrichtung hintereinander angeordnete Linsen mit zumindest einer Streulinse (18) und einer Sammellinse (20) umfassen und dass die Streulinse relativ zu der Sammellinse verstellbar angeordnet ist.

13. Steuerverfahren für eine Einrichtung für die laseroptische Augenchirurgie, insbesondere für eine Einrichtung nach einem der vorhergehenden Ansprüche, wobei die Einrichtung eine Quelle (10) gepulster Femtosekunden-Laserstrahlung, eine Mehrzahl im Strahlengang der Laserstrahlung hintereinander angeordneter Linsen, von denen wenigstens eine (18) in Richtung des Strahlengangs relativ zu anderen Linsen (20) verstellbar angeordnet ist, sowie eine Stellanordnung (24) zur Verstellung der wenigstens einen verstellbaren Linse (18) umfasst, wobei bei dem Verfahren auf Grundlage gespeicherter Topographiemessdaten eine Soll-Position für die verstellbare Linse (18) ermittelt wird und abhängig von der ermittelten Soll-Position ein Steuersignal für die Stellanordnung (24) erzeugt wird.

## Claims

1. Apparatus for laser-optical eye surgery, comprising a source (10) of pulsed femtosecond laser radiation and optical components (12, 14, 16) for guiding the laser radiation and focussing the same onto a treatment location on or in the eye, the optical components including a plurality of lenses (18, 20) arranged in succession in the beam path of the laser radiation, wherein at least one (18) of the lenses is arranged so as to be adjustable relative to other lenses in the direction of the beam path, **characterised in that** an actuating arrangement (24) is associated with the adjustable lens for its adjustment and **in that** for the purpose of controlling the actuating arrangement a control unit (26) is provided which is configured to access measured data concerning the topography of a surface of the eye and to control the actuating arrangement based on the measured surface topography.

2. Apparatus according to Claim 1, **characterised in that** the at least one adjustable lens (18) is part of beam-expansion optics (12) which are situated in the beam direction upstream of a scanning unit (14) scanning the laser radiation in a plane transverse to the beam direction.

3. Apparatus according to Claim 2, **characterised in that** the beam-expansion optics (12) at least include a diverging lens (18) and a converging lens (20) situated downstream thereof in the beam direction, the diverging lens being adjustable relative to the converging lens by means of the actuating arrangement (24).

4. Apparatus according to one of Claims 1 to 3, **characterised in that** the control unit (26) is set up to ascertain a nominal position for the adjustable lens (18) based on the measured surface topography and also based on a height distance of a desired location of action of the radiation in the eye from the topographically surveyed surface and to control the actuating arrangement (24) based on the ascertained nominal position.

5. Apparatus according to Claim 4, **characterised by** a measuring arrangement (32) which is set up to detect displacements of the height position of at least one reference location on or in the eye, the control unit (26) being set up to correct the nominal position of the adjustable lens (18) based on the detected current height position of the at least one reference location and to control the actuating arrangement (24) based on the corrected nominal position.

6. Apparatus according to Claim 4 or 5, **characterised by** a measuring arrangement (32) which is set up to detect movements of at least one reference location on or in the eye (28) in a plane transverse to the direction of the beam path, the control unit (26) being set up to correct the nominal position of the adjustable lens (18) based on the detected current transverse position of the at least one reference location and to control the actuating arrangement (24) based on the corrected nominal position.

7. Apparatus according to one of Claims 1 to 6, **characterised in that** the device is free from a contact lens to be placed onto the cornea and also free from mounting structures for such a contact lens.

8. Apparatus according to one of Claims 1 to 7, **characterised by** a measuring arrangement (32) which is set up to survey the corneal surface of the eye topographically.

9. Apparatus according to one of Claims 1 to 8, **characterised in that** the focus diameter of the laser radiation is not greater than about 10 µm, preferentially not greater than about 7 µm, and highly preferentially not greater than about 5 µm and **in that** the Rayleigh length of the laser radiation is not greater than about 20 µm, preferentially not greater than about 15 µm, and highly preferentially not greater than about 10 µm.

10. Apparatus according to one of Claims 1 to 9, **characterised in that** for the production of a two-dimensional incision in the cornea that is substantially parallel to the corneal surface by line scanning the control unit (26) is set up to provide to the actuating arrangement a control signal with approximate triangular characteristic and with varying triangle height.

11. Apparatus according to one of Claims 1 to 10, **characterised in that** for the production of a two-dimensional incision in the cornea that is substantially parallel to the corneal surface by spiral scanning the control unit (26) is set up to provide to the actuating arrangement a control signal of monotonically variable amplitude.

12. Apparatus according to one of Claims 1 to 11, **characterised in that** the optical components are constituted by beam-expansion optics (12), a scanning unit (14) arranged downstream of the beam-expansion optics in the direction of the beam path and serving for beam scanning in a plane transverse to the beam direction, and also focusing optics (16) arranged downstream of the scanning unit in the direction of the beam path, **in that** the beam-expansion optics (12) include several lenses arranged in succession in the beam direction and at least including a diverging lens (18) and a converging lens (20), and **in that** the diverging lens is arranged so as to be adjustable relative to the converging lens.

13. Control method for an apparatus for laser-optical eye surgery, optionally a device according to one of the preceding claims, wherein the device includes a source (10) of pulsed femtosecond laser radiation, a plurality of lenses arranged in succession in the beam path of the laser radiation, of which at least one (18) is arranged so as to be adjustable relative to other lenses (20) in the direction of the beam path, and also an actuating arrangement (24) for adjusting the at least one adjustable lens (18), wherein in the method a nominal position for the adjustable lens (18) is ascertained on the basis of stored topographical measured data and a control signal for the actuating arrangement (24) is generated based on the ascertained nominal position.

## Revendications

1. Dispositif pour la chirurgie oculaire au laser optique, comprenant une source (10) de rayonnement laser femtoseconde pulsé et des composants optiques (12, 14, 16) servant à guider le rayonnement laser et à focaliser ce dernier sur une zone de traitement située sur ou dans l'oeil, lesdits composants optiques comportant une pluralité de lentilles (18, 20) disposées les unes derrière les autres dans la trajectoire du rayon laser, au moins une (18) desdites lentilles étant disposée réglable par rapport aux autres lentilles en direction du trajet du rayon, **caractérisé en ce qu'**un dispositif de réglage (24) est associé à la lentille réglable pour le réglage de cette dernière et **en ce qu'**il est prévu, pour la commande du dispositif de réglage, une unité de commande (26) aménagée pour accéder à des données de mesure topographiques d'une surface oculaire et commander ledit dispositif de réglage en fonction de la topographie de la surface mesurée.

2. Dispositif selon la revendication 1, **caractérisé en ce que** ladite lentille (18) réglable fait partie intégrante d'une optique d'élargissement de rayon (12) située en direction du rayon laser, en avant d'une unité de déflexion (14) déviant le rayonnement laser dans un plan perpendiculaire à la direction du rayon laser.

3. Dispositif selon la revendication 2, **caractérisé en ce que** l'optique d'élargissement de rayon (12) présente au moins une lentille divergente (18) et une lentille convergente (20) située à l'arrière de celle-ci, en direction du rayon laser, la lentille divergente pouvant être réglée par rapport à la lentille convergente au moyen du dispositif de réglage (24).

4. Dispositif selon l'une des revendications 1 à 3, **caractérisé en ce que** l'unité de commande (26) est aménagée pour déterminer une position théorique pour la lentille réglable (18) en fonction de la topographie de la surface mesurée et de la distance verticale entre une zone où le rayonnement laser est appelé à agir dans l'oeil et la surface topographiquement mesurée, et pour commander le dispositif de réglage (24) en fonction de la position théorique déterminée.

5. Dispositif selon la revendication 4, **caractérisé par** un dispositif de mesure (32) aménagé pour détecter des déplacements de la position verticale d'au moins une zone de référence située sur ou dans l'oeil, l'unité de commande (26) étant aménagée pour corriger la position théorique de la lentille réglable (18) en fonction de la position verticale actuelle détectée de ladite zone de référence et commander le dispositif de réglage (24) en fonction de la position théorique corrigée.

6. Dispositif selon la revendication 4 ou 5, **caractérisé par** un dispositif de mesure (32) aménagé pour détecter les mouvements d'au moins une zone de référence située sur ou dans l'oeil (28) dans un plan perpendiculaire à la trajectoire du rayon laser, l'unité de commande (26) étant aménagée pour corriger la position théorique de la lentille réglable (18) en fonction de la position transversale actuelle détectée de ladite zone de référence et commander le dispositif de réglage (24) en fonction de la position théorique corrigée.

7. Dispositif selon l'une des revendications 1 à 6, **caractérisé en ce que** le dispositif est dépourvu de verre de contact à appliquer sur la cornée et dépourvu de formations permettant l'application d'un tel verre de contact.

8. Dispositif selon l'une des revendications 1 à 7, **caractérisé par** un dispositif de mesure (32) aménagé pour mesurer topographiquement la surface de la cornée de l'oeil.

9. Dispositif selon l'une des revendications 1 à 8, **caractérisé en ce que** le diamètre de foyer du rayonnement laser n'est pas supérieur à environ 10 µm, de préférence à environ 7 µm et encore plus préférentiellement à environ 5 µm, et **en ce que** la longueur de Rayleigh du rayonnement laser n'est pas supérieure à environ 20 µm, de préférence à environ 15 µm et encore plus préférentiellement à environ 10 µm.

10. Dispositif selon l'une des revendications 1 à 9, **caractérisé en ce que** l'unité de commande (26) est aménagée pour fournir au dispositif de réglage un signal de commande à caractéristique approximativement triangulaire et à hauteur triangulaire variable pour réaliser dans la cornée une coupe pour l'essentiel parallèle à la surface de la cornée par balayage linéaire.

11. Dispositif selon l'une des revendications 1 à 10, **caractérisé en ce que** l'unité de commande (26) est aménagée pour fournir au dispositif de réglage un signal de commande d'amplitude variable de manière monotone pour réaliser dans la cornée une coupe pour l'essentiel parallèle à la surface de la cornée par balayage en spirale.

12. Dispositif selon l'une des revendications 1 à 11, **caractérisé en ce que** les composants optiques forment une optique d'élargissement de rayon (12), une unité de déflexion (14) servant à dévier le rayon laser dans un plan perpendiculaire à la direction du rayon laser et disposée en direction du trajet du rayon, à l'arrière de l'optique d'élargissement de rayon et une optique de focalisation (16) disposée en direction du trajet du rayon à l'arrière de l'unité de déflexion, **en ce que** l'optique d'élargissement de rayon (12) comporte plusieurs lentilles disposées les unes derrière les autres en direction du rayon laser et comprenant au moins une lentille divergente (18) et une lentille convergente (20), et **en ce que** la lentille divergente est disposée réglable par rapport à la lentille convergente.

13. Procédé de commande pour un dispositif pour la chirurgie oculaire au laser optique, en particulier pour un dispositif selon l'une des revendications précédentes, ledit dispositif comprenant une source (10) de rayonnement laser femtoseconde pulsé, une pluralité de lentilles disposées les unes derrière les autres dans la trajectoire du rayon laser, dont au moins une (18) est disposée réglable par rapport aux autres lentilles (20) en direction du trajet du rayon, un dispositif de réglage (24) pour le réglage de ladite lentille réglable (18), lors duquel procédé est déterminée une position théorique pour la lentille réglable (18) sur la base de données de mesure topographiques enregistrées, et généré un signal de commande pour le dispositif de réglage (24) en fonction de la position théorique déterminée.
